# FASCICULE DE BREVET EUROPEEN

(11) **EP 3 119 433 B1**
(45) Date de publication et mention de la délivrance du brevet: **09.05.2018**
(21) Numéro de dépôt: 15711153.5
(22) Date de dépôt: 19.03.2015
(51) Int. Cl.: A61K 45/06, A61K 9/00, A61K 31/165, A61K 31/445, A61K 31/55, A61P 25/00, A61P 25/02, A61K 31/454, A61K 31/555, A61K 31/69

(54) **INHIBITEURS DE L'ACÉTYLCHOLINESTÉRASE D'ACTION CENTRALE POUR LA PRÉVENTION ET/OU LE TRAITEMENT DES NEUROPATHIES CHIMIO-INDUITES ET LEURS SYMPTÔMES, COMPOSITIONS, UTILISATIONS, MÉTHODES ET TROUSSE CORRESPONDANTES**
ZENTRAL WIRKENDE ACETYLCHOLINESTERASE-HEMMER ZUR PRÄVENTION UND/ODER BEHANDLUNG VON CHEMISCH INDUZIERTEN NEUROPATHIEN UND DEN SYMPTOMEN DAVON SOWIE ZUGEHÖRIGE ZUSAMMENSETZUNGEN, VERWENDUNGEN, VERFAHREN UND KIT
CENTRALLY ACTING ACETYLCHOLINESTERASE INHIBITORS FOR THE PREVENTION AND/OR TREATMENT OF CHEMICALLY INDUCED NEUROPATHIES AND THE SYMPTOMS THEREOF, AND CORRESPONDING COMPOSITIONS, USES, METHODS AND KIT

(30) Priorité: 20.03.2014 FR 1452348
(43) Date de publication de la demande: 25.01.2017
(73) Titulaire: Centre Hospitalier Universitaire de Clermont-Ferrand, 63003 Clermont-Ferrand (FR); Université d'Auvergne, 63001 Clermont-Ferrand (FR)
(72) Inventeur: ESCHALIER, Alain, F-63400 Chamalieres (FR); FERRIER, Jérémy, F-03500 Paray-sous-briailles (FR); BALAYSSAC, David, F-63122 Boissejour (FR); MARCHAND, Fabien, F-63000 Clermont-ferrand (FR)
(74) Mandataire: Vidon Brevets & Stratégie
(86) Numéro de dépôt international: PCT/EP2015/055836
(87) Numéro de publication internationale: WO 2015/140265

(56) Documents cités:
- WO-A1-2007/012154
- WO-A1-2010/019560
- WO-A2-2009/011412
- FR-A1- 2 957 077
- US-A1- 2006 264 455
- Kimura, M. Obata, H.: "Antihypersensitivity effects of donepezil in a rat model of neuropathic pain", 41st Annual Meeting of the Society-for-Neuroscience; Washington, DC, USA; November 12 -16, 2011 , 13 novembre 2011 (2011-11-13), XP002729486, Extrait de l'Internet: URL:http://www.abstractsonline.com/Plan/Vi ewAbstract.aspx?sKey=da447a65-6cf1-4b40-81 b5-e8da6a784d42&cKey=c455fdeb-eab0-4479-b7 ce-35d0a6c4906c&mKey={8334BE29-8911-4991-8 C31-32B32DD5E6C8} [extrait le 2014-09-09]
- SHAW EDWARD G ET AL: "Phase II study of donepezil in irradiated brain tumor patients: effect on cognitive function, mood, and quality of life.", JOURNAL OF CLINICAL ONCOLOGY : OFFICIAL JOURNAL OF THE AMERICAN SOCIETY OF CLINICAL ONCOLOGY 20 MAR 2006, vol. 24, no. 9, 20 mars 2006 (2006-03-20), pages 1415-1420, XP002729487, ISSN: 1527-7755
- AUTHIER NICOLAS ET AL: "Assessment of allodynia and hyperalgesia after cisplatin administration to rats", NEUROSCIENCE LETTERS, LIMERICK, IE, vol. 291, no. 2, 15 septembre 2000 (2000-09-15), pages 73-76, XP002599724, ISSN: 0304-3940, DOI: 10.1016/S0304-3940(00)01373-2
- MORIGUCHI SHIGEKI ET AL: "Modulation of N-methyl-D-aspartate receptors by donepezil in rat cortical neurons", JOURNAL OF PHARMACOLOGY AND EXPERIMENTAL THERAPEUTICS, vol. 315, no. 1, octobre 2005 (2005-10), pages 125-135, XP002729488, ISSN: 0022-3565

## Description

### 1. Domaine de l'invention

Le domaine de l'invention est celui de la prévention et/ou du traitement de(s) neuropathie(s) chimio-induite(s), particulièrement la prévention et/ou le traitement de(s) neuropathie(s) induites par les chimiothérapies. Les symptômes tels que la douleur ainsi que les autres troubles de la sensibilité associés à ces neuropathies pourront être ciblés. Plus précisément, l'invention concerne l'utilisation d'au moins un inhibiteur réversible de l'acétylcholinestérase d'action centrale pour la prévention et/ou le traitement des neuropathies induites par les chimiothérapies ; une composition comprenant ledit inhibiteur pour utilisation dans la prévention et/ou le traitement des neuropathies induites par les chimiothérapies.

### 2. Art antérieur

Le cancer est l'une des premières causes de mortalité et sa prévalence augmente avec l'âge de la population. En particulier, le cancer colorectal arrive au troisième rang des cancers les plus fréquemment diagnostiqués dans les pays industrialisés (source : Institut National du Cancer, mise à jour du 27/12/2012). L'un des traitements indiqués pour le cancer colorectal avancé est l'association d'un sel de platine (oxaliplatine) avec le 5-fluorouracile et l'acide folinique. Ce traitement est connu sous le nom de protocole FOLFOX4.

L'oxaliplatine (cis -[(1R, 2R)-1,2-cyclohexanediamine-N, N'] [oxaloto (2) - O, O'] platinum) est un sel de platine de troisième génération succédant au cisplatine et au carboplatine. Des autorisations de mise sur le marché ont été délivrées pour ce principe actif pour le traitement du cancer colorectal. Des études ont également été réalisées en rapport avec le traitement du cancer de l'ovaire et de l'estomac. Le cisplatine reste indiqué pour le traitement du cancer du poumon non à petites cellules, certains sarcomes et le cancer de l'ovaire. Le carboplatine est autorisé pour le traitement de cancers de l'ovaire, du poumon, du sein, de la vessie, de l'oesophage, de l'endomètre et des cancers ORL.

L'oxaliplatine présente l'avantage d'être moins néphrotoxique que le cisplatine et moins hématotoxique que le carboplatine. Cette molécule n'est pour autant pas dénuée d'effets indésirables.

Outre la fatigue, les nausées et les troubles digestifs classiquement occasionnés par les chimiothérapies, le principal facteur limitant de l'oxaliplatine est sa neurotoxicité, supérieure à celle observée avec les chimiothérapies à base de cisplatine et de carboplatine. Cela se caractérise notamment par une neurotoxicité aigüe constatée chez environ 90% des patients traités, c'est-à-dire une neurotoxicité survenant de quelques heures à quelques jours après chaque administration de l'oxaliplatine.

De manière plus précise, la neurotoxicité de l'oxaliplatine se manifeste, notamment lors de sa phase aigüe, par une neuropathie caractérisée par des paresthésies et une dysesthésie induites par le froid au niveau des mains, de la gorge, de la bouche et des joues, des engourdissements (Raymond et al., Ann Oncol, 1998, 9 (10) : 1053-1071) ainsi qu'une hypersensibilité mécanique et thermique (Binder et al, Eur J Cancer. 2007 Dec; 43(18):2658-63; Attal et al., Pain. 2009 Aug; 144(3):245-52).

Cette neurotoxicité revêt ensuite un caractère chronique au fur et à mesure du traitement. Les études cliniques ont démontré qu'environ 50% à 70% des patients développaient une neuropathie périphérique persistante et invalidante, similaire à celles observées avec les chimiothérapies à base de carboplatine et/ou de cisplatine. Ce type de neuropathies se traduit par une atteinte principalement sensitive, une persistance des paresthésies et des sensations d'engourdissement entre les cures de chimiothérapie, ainsi que des douleurs spontanées. Dans les cas les plus sévères, les patients développent une ataxie sensorielle accompagnée d'une perte de la sensibilité superficielle et profonde affectant le moindre geste du quotidien. Une étude récente (Park et al, Oncologist. 2011; 16(5):708-16) a par ailleurs démontré que la neuropathie devenait persistante chez environ 80% des patients concernés, et pouvait progresser pour s'installer bien après la fin d'un traitement à l'oxaliplatine et/ou au cisplatine.

La douleur neuropathique est un symptôme de la neuropathie, défini comme une douleur secondaire à une lésion ou un état pathologique affectant le système somatosensoriel. Elle est secondaire à des lésions périphériques (fibres nerveuses afférentes) ou centrales (centres nerveux impliqués dans la transmission et/ou la régulation du message douloureux, par exemple au niveau du thalamus).

Les étiologies de la douleur neuropathique sont diverses. Elles peuvent être causées par une lésion physique comme c'est le cas lors d'un accident, un acte chirurgical, une amputation (membre fantôme). Elles peuvent également être chimio-induites comme c'est le cas suite à un abus d'alcool, une intoxication aux métaux (arsenic ou thallium par exemple), l'exposition à un agent chimique environnemental (organophosphoré par exemple), un médicament, une toxine fongique ou bactérienne. Elles peuvent également faire suite à un état pathologique acquis ou héréditaire (par exemple une amylose, le syndrome de Guillain-Barré, une infection au VIH, une infection bactérienne, un zona, un herpès, un diabète, une maladie auto-immune et/ou une maladie de Fabry).

Les mécanismes physiopathologiques responsables des douleurs neuropathiques sont encore mal connus. Certaines études ont mis en lumière l'implication, entre autres, des récepteurs GABA, des transporteurs K⁺/Cl⁻, une perturbation de l'homéostasie du Cl⁻et/ou du co-transporteur KCC2 (Ferrini F et al, Neural Plast, 2013 : 429815 ; Asiedu MN et al, J. Pain, 2014 Jan 9), une diminution du taux de sérotonine (Sumpton JE et al, Handb Clin Neurol 2014; 119:513-27), l'implication du site de liaison à l'ATP des récepteurs P2X (Chataigneau et al, Front Cell Neurosci, 2013 (30) 7-273), de variation d'expression de divers canaux ioniques lors de neuropathie induite par l'oxaliplatine (Descoeur et al, Embo Molecular Medicine, 2011 3(5): 266-278), et/ou un dysfonctionnement de la barrière hémato-nerveuse (Lim TK et al, Pain. 2014 May; 155(5):954-67).

Ainsi, il existe une importante hétérogénéité au niveau des mécanismes moléculaires et cellulaires à l'origine des neuropathies. Ces mécanismes physiopathologiques sont parfois très différents en fonction de l'étiologie. Par exemple, il a été montré une implication différentielle de certains seconds messagers dans les neuropathies post-traumatiques comparées aux neuropathies chimio-induites (Aley et Levine, Neuroscience. 2002; 111(2):389-97). Ces deux types de neuropathies, bien que partageant des symptômes communs, doivent donc être considérés comme des syndromes bien distincts. Cette sous-catégorisation apparait donc primordiale et se vérifie également en termes de traitements pharmacologiques. En effet, bien que certaines molécules aient reçu une indication pour le traitement des douleurs neuropathiques post-traumatiques, aucune d'entre elles n'a démontré d'efficacité substantielle pour soulager les douleurs neuropathiques chimio-induites. C'est par exemple le cas de l'amitriptyline et de la gabapentine qui sont pourtant recommandées comme traitement de première ligne des douleurs neuropathiques post-traumatiques (Piano et al., Pain practice, 2014) mais qui n'ont apporté aucun soulagement chez les patients traités par l'oxaliplatine (Kautio et al., J. of pain and symptom management, 2008; Mitchell et al., Clin. Colorectal Cancer, 2006).

La neuropathie est donc un terme générique englobant différents symptômes non spécifiques, tout comme le terme « cancer » désigne la prolifération cellulaire anormale et pathologique au sein d'un tissu sain sans pour autant préjuger de la cause, des symptômes, de l'histologie et du traitement à administrer au patient.

Il est donc scientifiquement incorrect de mettre sur le même plan toutes les neuropathies (post-traumatiques, lésionnelles, chimio-induites) sans tenir compte de leur étiologie et des mécanismes biologiques mis en oeuvre. En d'autres termes, et à titre d'exemple, un unique symptôme comme l'hypersensibilité au froid ne peut pas être raisonnablement relié à un unique mécanisme biologique, une unique cause et donc à un unique traitement.

Comme énoncé précédemment, les douleurs neuropathiques d'origine traumatiques sont différentes des neuropathies chimio-induites et des douleurs associées aux neuropathies chimio-induites.

Par exemple, la majorité des antalgiques utilisés pour les douleurs neuropathiques (plutôt d'origine traumatique) ne fonctionnent pas ou peu pour les neuropathies chimio-induites hormis la duloxétine (Finnerup et al., Lancet Neurol. 2015 Jan ; S1474-4422(14)70251-0) ; Hershman et al., J Clin Oncol. 2014 Jun 20; 32(18):1941-67).

En d'autres termes, dans le cas des modèles animaux, même si on retrouve des symptômes communs (troubles sensitifs), la physiopathologie de ces neuropathies traumatiques (ligature du nerf sciatique), métaboliques (diabète) et toxiques (anticancéreux) est différente : expression ou non de protéines kinase, expression ou non des récepteurs NMDA... (Aley et Levine, Neuroscience. 2002; 111(2):389-97). Or, c'est le mécanisme mis en oeuvre qui conditionne le traitement, et non le symptôme.

Pour illustrer ce point par un exemple du quotidien, il est faux de relier une douleur au ventre à une unique cause (colique, intoxication, maladie auto-immune, cancer) et donc à un unique traitement (antispasmodique, antibiotique, immunosuppresseur, chimiothérapie).

En ce qui concerne les neuropathies (notamment les douleurs neuropathiques et troubles de la sensibilité associés) induites par les chimiothérapies telles celles aux sels de platine, il semblerait qu'elles soient liées, de manière directement dépendante, à la durée de la cure et à la dose administrée. Par conséquent, afin de limiter, voire de supprimer les symptômes des neuropathies induites par les traitements aux sels de platine, il est actuellement procédé à une adaptation posologique ; c'est-à-dire une diminution des doses administrées de sels de platine, un espacement des cures, voire l'interruption des cures. Or, cette adaptation posologique - bien que permettant dans certains cas de limiter, voire de prévenir les douleurs neuropathiques induites -compromet les chances de rémission et de survie du patient.

Outre les sels de platine, d'autres composés, médicamenteux ou non médicamenteux, entraînent des neuropathies. En particulier, certains médicaments utilisés pour les chimiothérapies comme le bortezomib, la thalidomide et les poisons du fuseau mitotique induisent également des neuropathies.

A titre d'exemple de composés non médicamenteux ayant une neurotoxicité, on peut citer l'alcool, l'arsenic, le thallium, les composés organophosphorés, etc.

Jusqu'à présent, les traitements s'attachent à réduire les symptômes un par un sans parvenir nécessairement à agir plus en amont pour les traiter de manière efficace et ciblée. Ainsi, il est parfois indiqué de prescrire des antidépresseurs tricycliques, de la gabapentine, de la prégabaline, une psychothérapie, l'éducation du patient pour gérer et vivre avec sa douleur. Cela revient à piocher dans la pharmacopée les médicaments qui pourraient soulager le patient, sans aucune certitude, ni critères objectifs.

### 3. Objectifs de l'invention

L'invention a notamment pour objectif de pallier ces inconvénients de l'art antérieur et de proposer une alternative thérapeutique à la simple adaptation posologique.

Plus précisément, l'invention a pour objectif, dans au moins un mode de réalisation, de fournir une composition permettant de prévenir et/ou d'éliminer, ou à tout le moins de réduire, les symptômes des neuropathies chimio-induites tels que la douleur et les troubles de la sensibilité.

L'invention a également pour objectif de fournir, dans au moins un mode de réalisation, de prévenir le développement des symptômes de la neuropathie induite par l'administration d'un composé anticancéreux neurotoxique.

En particulier, un objectif de l'invention est, dans au moins un mode de réalisation, de proposer une telle composition qui permette d'éliminer ou à tout le moins de réduire les symptômes de la neuropathie induite par l'administration de sels de platine.

### 4. Exposé de l'invention

Ces objectifs, ainsi que d'autres qui apparaîtront par la suite, sont atteints à l'aide d'une composition pour l'utilisation dans le traitement et/ou la prévention des neuropathies chimio-induites et leurs symptômes, ladite composition comprenant au moins un inhibiteur réversible de l'acétylcholinestérase d'action centrale.

On entend par « neuropathie chimio-induite » les neuropathies provoquées par l'administration ou l'exposition à un composé neurotoxique, ce composé pouvant être:
- soit un composé médicamenteux, en particulier un composé anticancéreux présentant une neurotoxicité;
- soit un composé non médicamenteux tels que l'alcool, les organophosphorés, les métaux lourds (Pb, As, Hg, TI), les toxines fongiques ou bactériennes, certains solvants comme le toluène, etc.

On entend par « composé neurotoxique » un composé agissant comme un poison sur le système nerveux. Les composés neurotoxiques agissent classiquement en perturbant, voire en bloquant, l'influx nerveux.

On entend par « inhibiteur réversible » une substance qui se lie de manière non covalente (liaison hydrogène, liaison ionique, interaction hydrophobe, etc.) à sa cible. Les inhibiteurs réversibles ne subissent pas de réaction chimique en se liant à leur cible et peuvent donc être facilement éliminés, contrairement aux inhibiteurs irréversibles qui forment un complexe stable avec leur cible de manière permanente.

On entend par « d'action centrale » tout composé capable de franchir la barrière hémato-encéphalique afin d'exercer son effet sur le système nerveux central (cerveau et moelle épinière).

Les inventeurs ont en effet découvert de manière étonnante que l'administration d'inhibiteurs réversibles de l'acétylcholinestérase d'action centrale - usuellement utilisés pour le traitement de démence comme la maladie d'Alzheimer - permettent de prévenir, d'atténuer, voire de traiter, les neuropathies chimio-induites et leurs symptômes tels que les troubles de la sensibilité et la douleur.

Comme évoqué précédemment, la neuropathie est une pathologie affectant au moins un nerf. Les symptômes de la neuropathie sont divers mais comprennent généralement des troubles de la sensibilité et des douleurs comme l'allodynie, l'hyperesthésie, la dysesthésie et l'ataxie. La douleur neuropathique est une douleur neurogène. A noter toutefois que la neuropathie ne s'accompagne pas nécessairement d'une douleur. Différents facteurs, activant différentes voies de signalisation, peuvent occasionner une telle neuropathie. Par conséquent, les traitements qui peuvent être proposés sont extrêmement divers et leur efficacité respective varie en fonction du mécanisme physiopathologique.

Le traitement de la neuropathie nécessite de comprendre le mécanisme biologique sous-jacent et plus précisément, celui mis en oeuvre par une cause particulière. Si les modèles de ligature du nerf sciatique chez le rat ont permis de faire avancer la compréhension des neuropathies post-traumatiques (amputation par exemple), ce modèle n'est pas transposable à toutes les réalités cliniques. Notamment, ce modèle ne permet pas d'apporter un éclairage quelconque aux polyneuropathies induites par un composé anticancéreux neurotoxique particulier.

De la même manière, on connaît le document US-2006/0264455-A1 qui décrit un modèle de douleur inflammatoire après une injection au formol. Le formol cause des lésions tissulaires et une douleur inflammatoire qui ne correspond à aucune situation clinique, si ce n'est une injection ou une ingestion de formol. Comme l'explique la revue de M. Barrot portant sur les différents tests et modèles utilisés pour simuler la douleur chez les rongeurs, l'injection au formol est un modèle de douleur inflammatoire et non un modèle de douleur neuropathique y compris chimio-induites (M. Barrot, Neurosciences 211 (2012) 39-50). En effet, la douleur neuropathique est une conséquence directe d'une lésion ou d'une pathologie affectant le système nerveux de manière primaire. A l'inverse, la douleur inflammatoire résulte d'une lésion ou d'une infection. Le système immunitaire libère des médiateurs chimiques qui peuvent endommager le système nerveux, de manière secondaire. Le modèle à l'injection de formol est donc un modèle de douleur inflammatoire à court-terme.

Par ailleurs, ce document indique que l'huperzine permet de traiter la douleur en inhibant les récepteurs NMDA. Or, les antagonistes des récepteurs NMDA sont connus pour leur propriétés antinociceptives dans le modèle de ligature du nerf sciatique (Mao et al., Brain Res. 1993 Mar 5, 605(1):164-8). Ces différents éléments illustrent bien la complexité du domaine et la complète impossibilité de raisonner de manière linéaire : neuropathie = 1 cause = 1 mécanisme = 1 traitement.

Parmi toute la palette de possibilités, les inventeurs ont découvert que les inhibiteurs réversibles de l'acétylcholinestérase d'action centrale permettent de prévenir, de réduire, voire d'éliminer, les symptômes des neuropathies chimio-induites, en particulier les douleurs et les troubles de la sensibilité.

Autrement dit, l'administration d'inhibiteurs réversibles de l'acétylcholinestérase d'action centrale permet de prévenir et/ou de réduire, voire d'éliminer, la neurotoxicité des composés anticancéreux neurotoxiques, aussi bien dans la phase chronique que dans la phase aigüe.

Dans ce cas, la composition selon l'invention présente un grand intérêt thérapeutique. Les inhibiteurs réversibles de l'acétylcholinestérase d'action centrale permettent en effet d'éviter que la neuropathie ne devienne chronique au fur et à mesure des cures de chimiothérapies. Par conséquent, le recours à de tels inhibiteurs doués d'effet préventif et curatif de la douleur neuropathique (voire d'autres troubles de la sensibilité) assurerait un confort très appréciable du patient tout en permettant de maintenir le rythme recommandé des cures, les posologies d'anticancéreux et en évitant d'interrompre le traitement des patients. Ainsi, les chances du patient face à la maladie, après traitement, seraient augmentées.

Selon l'invention, ledit inhibiteur réversible de l'acétylcholinestérase d'action centrale est choisi parmi le groupe comprenant le donépézil, un de ses sels pharmaceutiquement acceptable, un de ses énantiomères pharmaceutiquement acceptable, un de ses dérivés pharmaceutiquement acceptable ou leur mélange. Cet inhibiteur a l'avantage d'avoir été approuvé pour le traitement de démence notamment, chez l'humain. Ainsi, les preuves de son efficacité et de son relative innocuité chez l'homme ont déjà été rapportées. La dénomination IUPAC du donépézil est la suivante : (*RS*)-2-[(1-benzyl-4-piperidyl)methyl]-5,6-dimethoxy-2,3-dihydroinden-1-one). On entend par « sel pharmaceutiquement acceptable », les sels d'acides inorganiques ou organiques tels que les chlorhydrates, les bromhydrates, les sulfates, les nitrates, les phosphates, les formates, les acétates, les oxalates, les succinates, les maléates, les fumarates, les saccharates, ou des sels non toxiques de base comme le sodium, le potassium, le calcium, le magnésium, le zinc.

De manière davantage préférée ledit inhibiteur est un sel de donépézil ; de manière encore davantage préférée ledit inhibiteur est du chlorhydrate de donépézil. Le donépézil amène peu d'effets indésirables, ce qui est appréciable pour un patient qui par exemple doit déjà supporter les effets indésirables de la chimiothérapie anticancéreuse. Le chlorhydrate de donépézil est actuellement commercialisé sous la dénomination commerciale Aricept™ par les laboratoires Pfizer (Etats-Unis).

Avantageusement, la composition selon l'invention est utilisée pour le traitement et/ou la prévention des symptômes des neuropathies induites par un composé anticancéreux choisi parmi un sel de platine, le bortézomib, la thalidomide, un poison du fuseau mitotique ou leur mélange, ladite composition comprenant au moins un inhibiteur réversible de l'acétylcholinestérase d'action centrale tel que défini plus haut. De manière davantage préférée, ledit composé anticancéreux est un sel de platine choisi parmi le carboplatine, le cisplatine, l'oxaliplatine et leur mélange.

En effet, les inventeurs ont découvert qu'administrer des inhibiteurs réversibles de l'acétylcholinestérase d'action centrale en parallèle et/ou en amont d'une chimiothérapie aux sels de platine permet de prévenir et/ou traiter le développement de la neuropathie associée.

Les sels de platine présentent l'inconvénient d'entraîner de nombreux effets secondaires qui nécessitent l'adaptation posologique voire l'arrêt du traitement, compromettant de fait les chances du patient. Les inventeurs ont découvert que l'administration de donepezil, en particulier du chlorhydrate de donepezil, permettait de réduire les signes de la neuropathie induite par ces composés neurotoxiques.

Jusqu'à présent, les patients sous chimiothérapie avec un sel de platine et développant une neuropathie chimio-induite sont pris en charge par des traitements conventionnels, typiquement utilisés dans les douleurs neuropathiques traumatiques et avec très peu de résultats (Finnerup et al., Lancet Neurol. 2015 ; Hershman et al., J Clin Oncol. 2014). Et pour cause, cette approche thérapeutique ne tient pas compte de la spécificité du mécanisme en cause dans la neurotoxicité des sels de platine. Les inventeurs, par leur invention, offrent donc une alternative thérapeutique efficace aux patients sous chimiothérapie aux sels de platine, en particulier à l'oxaliplatine.

Ledit composé anticancéreux peut, également ou alternativement, être choisi parmi les poisons du fuseau mitotique. Cette classe d'anticancéreux bloque la division cellulaire des cellules cancéreuses en perturbant le fuseau mitotique et notamment en bloquant la polymérisation ou dépolymérisation des microtubules. Les cellules cancéreuses se caractérisent des cellules non cancéreuses en ce qu'elles prolifèrent beaucoup plus rapidement, la fréquence des mitoses étant donc plus élevée que celle des cellules non cancéreuses. L'utilisation de poisons du fuseau mitotique permet donc de cibler plus spécifiquement les cellules cancéreuses que les cellules non cancéreuses.

En particulier, le poison du fuseau mitotique peut être choisi parmi les composés de la classe des taxanes, les composés de la classe des épothilones, les alcaloïdes de la pervenche de Madagascar ou leur mélange.

Ledit composé anticancéreux neurotoxique peut, également ou alternativement, être choisi parmi les composés de la famille des épothilones. Les épothilones sont une classe d'anticancéreux agissant comme les composés de la famille des taxanes mais en étant plus efficaces et pourvoyeurs de moins d'effets indésirables. Les épothilones se fixent sur l'hétérodimère αβ - tubuline et bloquent ainsi la polymérisation des microtubules.

Ledit composé anticancéreux neurotoxique peut, également ou alternativement, être choisi parmi les composés de la famille des taxanes ; de préférence parmi le groupe comprenant le paclitaxel, le docetaxel et leur mélange. Le paclitaxel (Taxol™, Abraxane™) est un antimitotique utilisé notamment pour le traitement du cancer du sein, des ovaires et du poumon. Le docetaxel (Taxotere™) est utilisé notamment pour le traitement du cancer du sein.

Ledit composé peut, également ou alternativement, être choisi parmi les alcaloïdes de la pervenche de Madagascar; de préférence parmi le groupe comprenant la vincristine, la vinblastine et leur mélange. La vincristine (commercialisée sous le nom Oncovin) est un antimitotique utilisé notamment pour le traitement du cancer du poumon, du lymphome non Hodgkinien et du lymphome de Hodgkin, de la leucémie et du myélome multiple. La vinblastine (commercialisée sous les noms Velban et Alkaban-AQ) est utilisée dans le traitement des cancers de l'ovaire, de la vessie, du rein, du sein, du testicule, des lymphomes.

Ledit composé peut, également ou alternativement, être choisi parmi le bortezomib, la thalidomide et leur mélange. Le bortezomib (VeIcade®) est un inhibiteur du protéasome, indiqué notamment pour le traitement du myélome multiple. Le thalidomide (Celgène®, Immunoprin®, Thalidomid®) est utilisé notamment pour le traitement du myélome multiple.

Dans un mode de réalisation, la composition selon l'invention comprend entre 1 mg et 25 mg, de préférence entre 6 mg et 12 mg, de manière davantage préférée entre 5 à 10 mg, de chlorhydrate de donépézil. Il est à noter que 10 mg de chlorhydrate de donépézil correspondent à 9,12 mg de donépézil.

La dose administrée peut varier en fonction du poids, de l'âge et de la taille du patient ainsi qu'en fonction de la méthode d'administration, et de la forme galénique.

La composition selon l'invention peut être administrée par voie entérale (voie orale, voie perlinguale, voie rectale) ou par voie parentérale (injection intramusculaire, injection intraveineuse ou injection sous-cutanée).

Dans un mode de réalisation avantageux, la composition est administrée par voie orale. Ce mode d'administration est à préférer car il est plus pratique et plus simple pour le patient qu'une solution injectable. La composition peut alors être sous forme de comprimé pelliculé, de gélule, de dragée, de comprimé effervescent ou dispersible, ou de solution buvable tel un sirop, une solution, une suspension.

La composition selon l'invention peut également être administrée sous forme de comprimé à libération prolongée ou retardée.

Lorsque la composition selon l'invention est sous forme injectable, elle peut être sous forme d'une solution, d'une émulsion ou d'une suspension.

Dans un mode de réalisation préféré, la composition selon l'invention est administrée avant l'administration des composés anticancéreux neurotoxiques. Cette option permet de potentialiser les effets de l'inhibiteur réversible de l'acétylcholinestérase d'action centrale en lui laissant le temps d'agir et en évitant que la neuropathie et/ou les douleurs neuropathiques associées ne se développent. Les inventeurs ont en effet constaté qu'il est plus efficace d'empêcher la douleur de se développer et de s'installer plutôt que de la traiter une fois les premiers signes apparus. L'administration prophylactique d'au moins un inhibiteur réversible de l'acétylcholinestérase d'action centrale, avant l'administration d'au moins un composé anticancéreux neurotoxique, notamment un sel de platine, permet d'éviter le développement de la douleur neuropathique.

Les composés anticancéreux neurotoxiques sont tels que définis ci-avant.

Avantageusement, la composition selon l'invention est d'abord administrée à une dose de 5 mg/jour pendant une durée de 4 à 6 semaines, puis à une dose de 10 mg/jour pendant une durée de 4 à 6 semaines.

Il peut être envisagé d'augmenter progressivement la dose de la composition selon l'invention par palier croissant de 5 mg/jour, chaque palier pouvant durer entre 2 et 4 semaines, de sorte que le patient s'habitue progressivement à la composition, jusqu'à atteindre une dose maximale de 10 mg/jour, voire de 25 mg/jour.

Dans un mode de réalisation intéressant, la composition selon l'invention est administrée à heure régulière.

Selon l'invention, la composition comprend en outre au moins un adjuvant pharmaceutiquement acceptable. Des exemples d'adjuvant sont l'eau, des conservateurs, des antioxydants, des colorants, des solvants, des transporteurs, des stabilisants, des épaississants, des lubrifiants, des parfums ou des excipients permettant d'augmenter l'action de l'inhibiteur réversible de l'acétylcholinestérase d'action centrale, son absorption, son métabolisme et/ou son excrétion.

Un autre objet de l'invention concerne l'utilisation d'au moins un inhibiteur réversible de l'acétylcholinestérase d'action centrale pour le traitement et/ou la prévention de neuropathies chimio-induites et de leurs symptômes (notamment les douleurs neuropathiques et troubles de la sensibilité associés).

De telles neuropathies chimio-induites peuvent être induites par l'administration d'un composé anticancéreux neurotoxique. Ledit inhibiteur réversible de l'acétylcholinestérase d'action centrale et ledit composé neurotoxique sont tels que définis ci-avant.

Ledit inhibiteur réversible de l'acétylcholinestérase d'action centrale peut être choisi parmi le groupe comprenant le donépézil, un de ses sels pharmaceutiquement acceptable, un de ses énantiomères pharmaceutiquement acceptable, un de ses dérivés pharmaceutiquement acceptable ou leur mélange.

De manière particulièrement avantageuse, l'invention concerne l'utilisation d'au moins un inhibiteur réversible de l'acétylcholinestérase d'action centrale pour la prévention et/ou le traitement des neuropathies et leurs symptômes (notamment les douleurs neuropathiques et troubles de la sensibilité associés) induites par un composé anticancéreux neurotoxique choisi parmi un sel de platine, un composé de la famille des taxanes, un alcaloïde de la pervenche de Madagascar, le bortézomib, la thalidomide ou leur mélange.

Les inhibiteurs réversibles de l'acétylcholinestérase et les composés anticancéreux neurotoxiques sont tels que définis ci-avant.

Dans un mode de réalisation intéressant, l'invention concerne l'utilisation d'un sel de donépézil, de préférence le chlorhydrate de donépézil, pour la prévention et/ou le traitement des symptômes des neuropathies (notamment les douleurs neuropathiques et troubles de la sensibilité associés) induites par l'administration d'au moins un sel de platine.

Le sel de platine peut être choisi parmi le carboplatine, le cisplatine, l'oxaliplatine et leur mélange.

De préférence, ledit inhibiteur réversible de l'acétylcholinestérase d'action centrale est administré au patient à heure régulière.

Dans un mode de réalisation intéressant selon l'invention, l'étape d'administration dudit inhibiteur réversible de l'acétylcholinestérase d'action centrale se fait par paliers successifs de 4 à 6 semaines afin d'augmenter la dose de 5 mg par palier. En d'autres termes, l'étape d'administration dudit inhibiteur réversible de l'acétylcholinestérase comprend :
- une première sous étape durant 2 à 4 semaines pendant laquelle le patient reçoit une dose de 5 mg/jour ; et
- une seconde sous étape pendant laquelle le patient reçoit une dose de 10 mg/jour.

De préférence, la seconde sous étape dure entre 4 et 6 semaines.

L'étape d'administration dudit inhibiteur réversible de l'acétylcholinestérase d'action centrale peut comprendre au moins deux sous étapes durant chacune 4 à 6 semaines, la dose quotidienne d'au moins un inhibiteur réversible de l'acétylcholinestérase étant maintenue constante pendant la durée de chaque palier et étant augmentée de 5 mg/jour au terme de chacun des paliers.

Un autre objet de l'invention concerne une trousse, désignée sous le terme de « kit » en anglais, comprenant au moins un composé anticancéreux neurotoxique choisi parmi le groupe comprenant un sel de platine, le bortézomib, la thalidomide, un poison du fuseau mitotique ou leur mélange ; et l' inhibiteur réversible de l'acétylcholinestérase d'action centrale donépézil. Les poisons du fuseau mitotique sont tels que définis ci-avant.

Ledit inhibiteur réversible de l'acétylcholinestérase d'action centrale et ledit composé anticancéreux sont tels que définis ci-avant.

### 5. Liste des figures

D'autres caractéristiques et avantages de l'invention apparaîtront plus clairement à la lecture de la description suivante d'un mode de réalisation préférentiel, donné à titre de simple exemple illustratif et non limitatif, et des dessins annexés, parmi lesquels :
- la figure 1 présente un graphique illustrant l'effet d'une administration de donépézil (5 mg/kg, voie orale) sur l'allodynie mécanique suite à une administration chronique d'oxaliplatine chez le rat;
- la figure 2 présente un graphique représentant les résultats d'une administration de donépézil (5 mg/kg, voie orale) sur l'allodynie au froid suite à une administration chronique d'oxaliplatine chez le rat;
- la figure 3 présente un graphique illustrant l'effet préventif de l'administration chronique de donépézil (5 mg/kg, 2 fois par jour, voie orale) sur l'allodynie mécanique suite à l'administration chronique d'oxaliplatine chez le rat;
- la figure 4 présente un graphique démontrant l'effet préventif de l'administration chronique de donépézil (5 mg/kg, 2 fois par jour, voie orale) sur l'allodynie au froid suite à l'administration chronique d'oxaliplatine ;
- la figure 5 présente un graphique illustrant l'effet d'une administration de donépézil (5 mg/kg, voie orale) sur l'anxiété chez des rats traités ou non par l'oxaliplatine.

### 6. Description d'un mode de réalisation de l'invention

Le principe général de l'invention repose sur l'administration d'au moins un inhibiteur réversible de l'acétylcholinestérase d'action centrale pour prévenir et/ou traiter les neuropathies chimio-induites et leurs symptômes.

En particulier, l'invention comprend, dans un de ses aspects particulièrement intéressant, l'administration d'au moins un inhibiteur réversible de l'acétylcholinestérase d'action centrale en amont et/ou en parallèle de l'administration d'un composé anticancéreux neurotoxique choisi parmi un sel de platine, le bortézomib, la thalidomide, un poison du fuseau mitotique tels qu' un composé de la famille des taxanes ou un composé de la famille des épothilones, un alcaloïde de la pervenche de Madagascar, ou leur mélange.

Les sels de platine tels que l'oxaliplatine, le carboplatine et le cisplatine sont actuellement utilisés pour le traitement de certaines formes de cancer. Ils présentent tous une neurotoxicité plus ou moins importante en fonction des molécules, l'oxaliplatine étant le plus neurotoxique des sels de platine. Cette neurotoxicité se manifeste de manière aigüe suite à chaque administration d'oxaliplatine au patient, et devient chronique au fur et à mesure que le patient reçoit des doses de sels de platines (cisplatine, carboplatine et oxaliplatine). Cette neurotoxicité se manifeste par l'apparition de signes rencontrés dans les douleurs neuropathiques comme des sensations de brûlures, de fourmillements, un engourdissement, l'allodynie, la paresthésie qui persistent bien après l'arrêt du traitement. Les inventeurs ont observé de manière surprenante que l'administration d'une dose quotidienne d'inhibiteur réversible de l'acétylcholinestérase d'action centrale en parallèle d'une injection de sels de platine permettait de réduire la douleur neuropathique causée par ces sels, et d'empêcher le développement d'une douleur chronique. Ils ont également constaté qu'administrer un inhibiteur réversible de l'acétylcholinestérase d'action centrale avant d'administrer un sel de platine permettait de réduire davantage la douleur neuropathique voire de la prévenir. Les avantages de l'invention sont démontrés par les essais comparatifs suivants.

### 6.1. Protocole expérimental

### 6.1.1. Conditions expérimentales

Comme indiqué précédemment, l'oxaliplatine est un sel de platine de troisième génération qui succède au carboplatine et au cisplatine. L'oxaliplatine étant le plus neurotoxique de ces sels, les expériences ont été menées en utilisant l'oxaliplatine sur des rats mâles Sprague - Dawley.

L'oxaliplatine (Leancare, Royaume -uni) a été dissous dans une solution de glucose 5% à la concentration finale de 2 mg/ml afin d'obtenir un volume injectable de 1 mL/kg. La solution d'oxaliplatine est injectée 2 fois par semaine aux animaux par voie intraveineuse pendant une durée de 4,5 semaines (dose cumulée 18 mg/kg). Les animaux du groupe contrôle ont reçu une injection de glucose 5% par la même voie, à la même fréquence et au même volume.

Le chlorhydrate de donépézil est utilisé comme exemple d'inhibiteur réversible de l'acétylcholinestérase d'action centrale (Aricept™ 10 mg, Pfizer, Etats-Unis). Pour simplifier l'explication, le chlorhydrate de donépézil sera nommé dans la suite des exemples sous le terme de « donépézil ». Il se présente sous la forme de comprimés pelliculés. Ces comprimés ont été réduits en fine poudre puis mis en suspension dans de l'eau contenant 0,5% de carboxyméthylcellulose (Sigma-Aldrich, France) afin d'obtenir une solution à 4 mg/ml. Le donépézil a été administré par voie orale à la dose de 5 mg/kg. Les animaux du groupe contrôle ont simplement reçu une administration de carboxyméthylcellulose 0,5% par la même voie, au même volume et à la même fréquence.

Différents groupes d'animaux ont été créés afin de comparer les conditions :
- un groupe contrôle de 8 rats ne recevant que les solutions contrôle (glucose 5% et carboxyméthylcellulose 0,5%) (groupe Sham);
- un groupe de 8 rats ayant reçu une solution d'oxaliplatine et une solution de carboxyméthylcellulose 0,5% (groupe O);
- un groupe de 8 rats ayant reçu une solution de glucose 5% et une solution de donépézil (groupe D);
- un groupe de 8 rats ayant reçu une solution d'oxaliplatine et une solution de donépézil (groupe O-D).

### 6.1.2. Tests comportementaux

L'allodynie, au froid ou mécanique, correspond à une sensation de douleur ressentie suite à un stimulus normalement indolore chez un individu bien portant. Ce phénomène est caractéristique d'une douleur neuropathique. Différents tests comportementaux standardisés ont été menés pour évaluer la douleur neuropathique sur les animaux.

### Test de Von Frey : allodynie mécanique

L'appareil électronique de Von Frey (Chaplan et al., 1994) comporte un manche portatif constitué d'une pointe en plastique reliée à un capteur de force. Les rats sont placés dans des boites en plexiglas, disposées sur un grillage surélevé. Après une période d'habituation des rats, on applique la pointe perpendiculairement au centre des 5 coussinets plantaire de chaque patte postérieure. La pression appliquée est augmentée progressivement jusqu'à provoquer un réflexe de retrait de la patte. L'intensité du stimulus, la pression maximale appliquée (exprimée en grammes) est enregistrée par l'appareil lors du retrait de la patte.

### Test d'interaction sociale : anxiété

Ce test se déroule dans une arène équipée d'une caméra dans laquelle deux animaux sont mis en présence pendant 10 minutes. L'objectif de ce test est de mesurer le temps d'interaction entre les animaux. Les deux animaux étudiés font toujours partie du même groupe de condition expérimentale pour éviter que le comportement d'un rat n'influe sur l'autre. Les rats interagissent entre eux par reniflage, toilettage, poursuite... Une augmentation des interactions indique un état non anxieux du rat tandis qu'une diminution et une tendance à la prostration indique un état anxieux de l'animal. Au début de l'expérience, les animaux sont placés à des coins opposés de l'arène et le manipulateur quitte la pièce. L'arène est nettoyée entre deux expériences pour éviter que les odeurs des rats précédents ne perturbent les résultats.

### Test d'immersion de la queue : allodynie au froid

La queue des rats est immergée dans un bain d'eau froide (10°C). L'immersion est maintenue jusqu'à ce que l'animal retire sa queue. La latence du retrait est chronométrée. Chaque mesure est effectuée en triplicat.

### 6.2. Résultats

Les résultats ont été analysés par la méthode ANOVA à mesures répétées (2 facteurs).

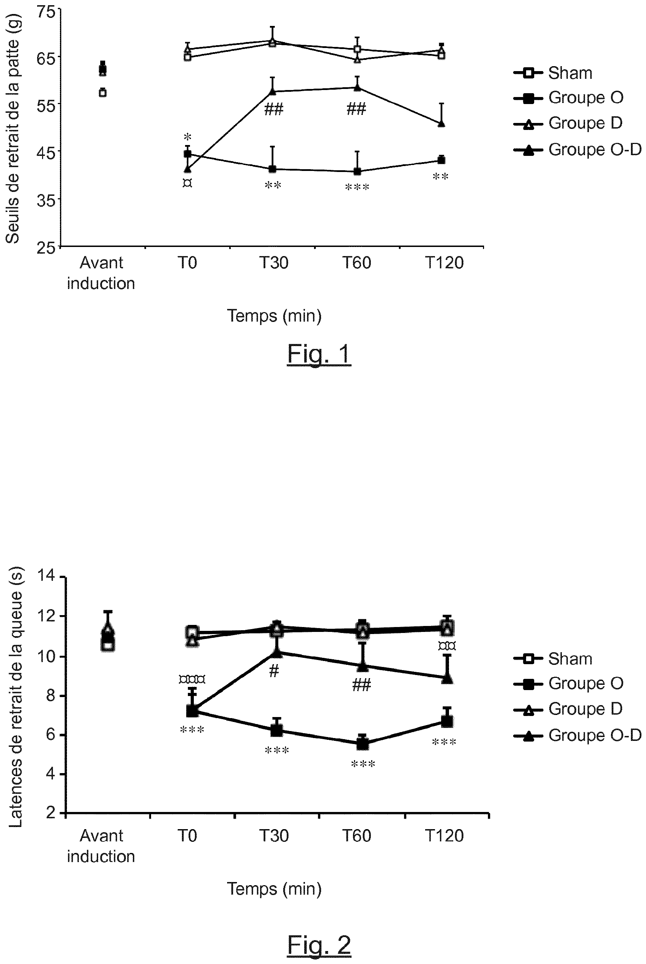

Sur les figures 1 à 6, les étoiles indiquent la significativité des résultats entre le groupe O et le groupe Sham selon l'échelle suivante :
- * = p<0,05 ;
- ** = p<0,01 ;
- *** = p<0,001.

Les cercles indiquent la significativité des résultats entre le groupe O et le groupe D selon l'échelle suivante :
- ¤ = p<0,05 ;
- ¤¤ = p<0,01 ;
- ¤¤¤ = p<0,001.

Les dièses indiquent la significativité des résultats entre le groupe O et le groupe O-D selon l'échelle suivante :
- # = p<0,05 ;
- ## = p<0,01 ;
- ### = p<0,001.

### 6.2.1. Effet d'une prise de donépézil sur la neuropathie induite par l'oxaliplatine

Les effets d'une administration d'une dose de donépézil de 5mg/kg sur l'allodynie mécanique statique et au froid ont été étudiés. Pour ces expériences, le donépézil a été administré aux rats à la fin du protocole d'injection de l'oxaliplatine, soit 31 jours après la première administration d'oxaliplatine, afin d'évaluer l'effet curatif du donépézil. Les essais ont été conduits sur une durée de 3 heures, sur les 4 groupes de rats. Le temps t0 correspond au moment de l'injection. Les résultats sont présentés dans les figures 1 et 2.

La figure 1 présente les résultats de l'expérience sur l'allodynie statique, évaluée grâce au test de Von Frey électronique. Comme on peut le constater, les animaux traités par l'oxaliplatine (groupe O) ont montré une diminution du seuil de retrait de la patte par rapport au groupe Sham. En d'autres termes, les animaux du groupe O ressentent une douleur à plus faible stimulation par rapport au groupe Sham. Ceci est cohérent avec les données cliniques récoltées chez l'humain. En revanche, on constate que les animaux du groupe O-D présentent une amélioration significative du seuil de retrait de la patte au bout de 30 min qui perdure jusqu'à 60 min après l'administration de donépézil.

La figure 2 présente les résultats de l'expérience sur l'allodynie au froid, évaluée grâce au test d'immersion de la queue dans de l'eau à 10°C. Les observations sont similaires à celles de la figure 1. Le groupe O présente un seuil de retrait de la queue significativement plus bas que le groupe Sham, ce qui signifie que les rats traités à l'oxaliplatine seul ont une sensibilité au froid accrue par rapport aux rats traités par les véhicules. Les résultats du groupe O-D démontrent que l'administration de donépézil permet aux rats d'augmenter leur tolérance au froid, ou autrement dit de diminuer l'allodynie au froid, au bout de 30 minutes et jusqu'à 120 minutes après la fin de l'administration.

En conclusion, ces essais démontrent que l'administration d'un inhibiteur réversible de l'acétylcholinestérase d'action centrale permet de réduire considérablement les symptômes de la douleur neuropathique induite par une exposition aux sels de platine. L'inhibiteur réversible de l'acétylcholinestérase d'action centrale permet d'inverser les signes de la douleur neuropathique en seulement 30 minutes. Ces résultats démontrent que l'administration d'un inhibiteur réversible de l'acétycholinestérase d'action centrale permet de supprimer la douleur neuropathique induite par l'oxaliplatine une fois installée.

### 6.2.2. Effet préventif d'une administration répétée de donépézil sur la neuropathie induite par l'oxaliplatine

L'expérience du point 6.2.2 a été menée sur le même modèle animal reproduisant la neuropathie induite par l'oxaliplatine, si ce n'est que l'étude a été menée entre J0 et J28. Les rats ont reçu une dose de 5 mg/kg de donépézil deux fois par jour pendant 2 semaines (du jour 14 au jour 28). Le traitement au donépézil a été instauré avant l'apparition des symptômes d'allodynie tactile et thermique afin d'évaluer son effet préventif.

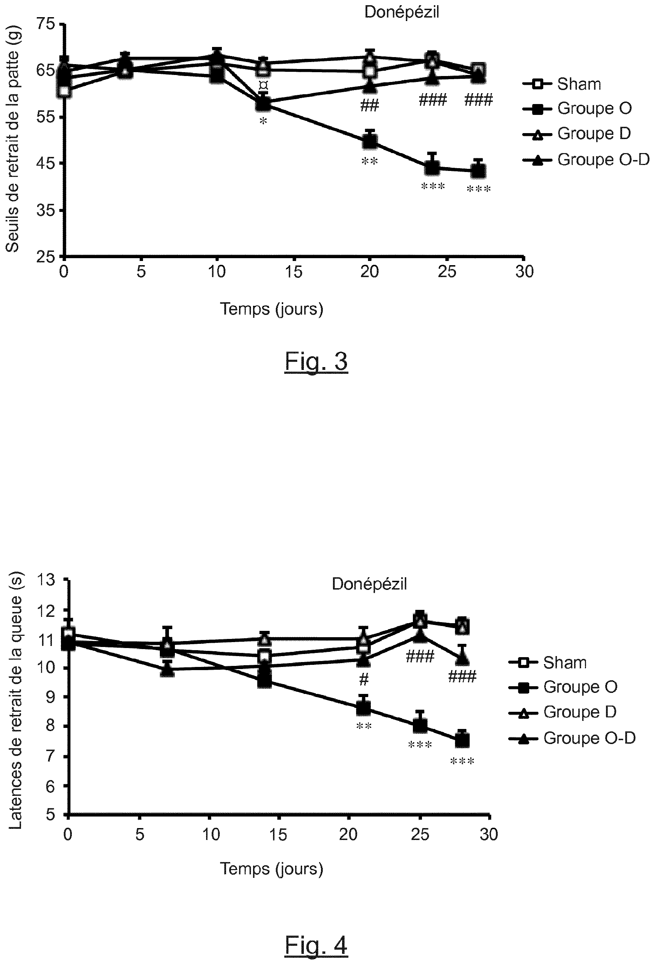

La figure 3 présente les résultats sur l'étude de l'allodynie mécanique, évaluée par le test de Von Frey. La figure 4 présente les résultats de l'effet d'une administration chronique de donépézil sur l'allodynie au froid, évaluée par le test d'immersion de la queue.

Comme on peut l'observer sur les figures 3 et 4, on constate que le groupe O présente une sensibilité accrue à la stimulation mécanique (figure 3) et au froid (figure 4) par rapport au groupe Sham. Cette observation est cohérente avec les résultats des essais de l'expérience 6.2.1 et les données cliniques chez l'homme.

En revanche, les figures 3 et 4 démontrent que les animaux traités par le donépézil de manière chronique (groupe O-D) ont des seuils de douleur significativement plus élevés que ceux des animaux traités par l'oxaliplatine seul (groupe O). Fait intéressant : les animaux du groupe O-D présentent des seuils de réponse au froid et à une stimulation mécanique similaires par rapport aux groupes Sham et D. Ceci démontre qu'une administration chronique d'un inhibiteur réversible de l'acétylcholinestérase d'action centrale permet de prévenir le développement de la douleur neuropathique induite par les sels de platine, et ce jusqu'à la fin de l'étude.

### 6.2.3. Effet d'une administration de donépézil sur la neuropathie induite par l'oxaliplatine : évaluation de l'anxiété

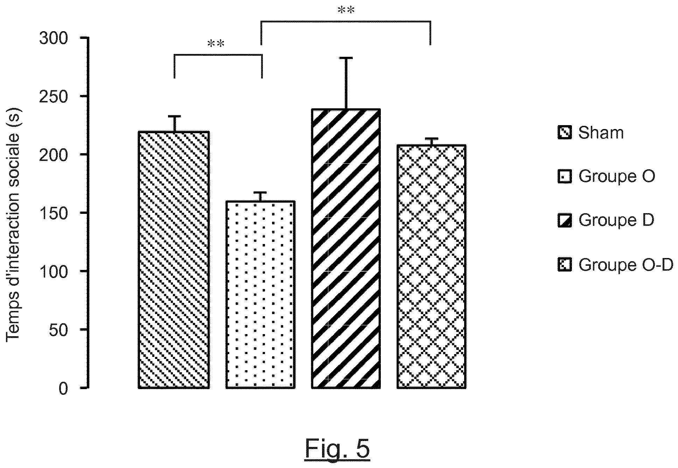

L'effet du donépézil sur l'anxiété a été étudié sur les 4 groupes par le test d'interaction sociale. Le test a été réalisé à la fin du protocole d'administration de l'oxaliplatine, soit au jour 31. Une dose de 5 mg/kg a été administrée aux animaux des groupes D et O-D 30 minutes avant le test. Les résultats du test d'interaction sociale sont présentés sur la figure 5. Plus le temps d'interaction sociale est faible, plus l'animal est anxieux.

Comme on peut l'observer, les animaux du groupe O sont significativement plus anxieux que les animaux du groupe Sham. Les animaux du groupe D ont un niveau d'anxiété similaire à ceux du groupe Sham, la différence n'étant pas significative. En revanche, les animaux du groupe O-D ont un niveau d'anxiété équivalent à ceux du groupe Sham et significativement moindre à ceux du groupe O. En d'autres termes, l'administration d'un inhibiteur de l'acétylcholinestérase d'action centrale permet de réduire l'anxiété associée aux douleurs neuropathiques induites par une exposition aux sels de platine.

### 7. Conclusion

Comme cela a été démontré, les sels de platine, et l'oxaliplatine en particulier, induisent généralement une douleur neuropathique chez les animaux traités qui se manifeste notamment par une allodynie mécanique, une allodynie au froid ; elle est accompagnée d'un niveau d'anxiété significativement plus élevé que chez les sujets non traités. L'administration d'au moins un inhibiteur réversible de l'acétylcholinestérase d'action centrale après l'administration d'au moins un sel de platine permet de réduire les symptômes de la douleur neuropathique, comme cela a été démontré au point 6.2.1. Une telle administration de manière chronique permet également de prévenir le développement d'une douleur neuropathique, comme cela a été prouvé au point 6.2.2. Enfin, l'administration d'au moins un inhibiteur réversible de l'acétylcholinestérase d'action centrale permet de réduire également l'anxiété du patient souffrant de douleur neuropathique.

Par conséquent, la composition selon l'invention permet de réduire et prévenir les manifestations sensitives, et particulièrement la douleur, liées aux neuropathies induites par les sels de platine. Cela est intéressant car les cures de chimiothérapies aux sels de platine sont planifiées. Il est donc possible d'administrer au moins un inhibiteur réversible de l'acétylcholinestérase de manière préventive, avant ou pendant la cure de chimiothérapie aux sels de platine, afin d'empêcher le développement de la douleur neuropathique.

Les inventeurs proposent donc une alternative thérapeutique efficace et ciblée aux traitements actuels concentrés sur l'atténuation des symptômes.

## Revendications

1. Composition pour utilisation dans le traitement et/ou la prévention des neuropathies chimio-induites et leurs symptômes, ladite composition comprenant au moins un inhibiteur réversible de l'acétylcholinestérase d'action centrale choisi parmi le groupe comprenant le donépézil, un de ses sels pharmaceutiquement acceptable, un de ses énantiomères pharmaceutiquement acceptable, et ladite neuropathie chimio-induite étant une neuropathie induite par un composé anticancéreux choisi parmi un sel de platine, le bortézomib, la thalidomide, un poison du fuseau mitotique ou leur mélange.

2. Composition pour utilisation selon la revendication 1 dans laquelle ledit inhibiteur est du chlorhydrate de donépézil.

3. Composition pour utilisation selon la revendication 1 ou 2 dans laquelle ledit composé anticancéreux neurotoxique est choisi parmi les sels de platine ; de préférence parmi le groupe comprenant le cisplatine, le carboplatine, l'oxaliplatine et leur mélange.

4. Composition pour utilisation selon la revendication 1 ou 2 dans laquelle ledit composé anticancéreux neurotoxique est un poison du fuseau mitotique choisi parmi les alcaloïdes de la pervenche de Madagascar, les composés de la famille des épothilones, les composés de la famille des taxanes ou leur mélange.

5. Composition pour utilisation selon la revendication 4 dans laquelle ledit composé anticancéreux neurotoxique est choisi parmi les alcaloïdes de la pervenche de Madagascar; de préférence parmi la vincristine, ou leur mélange.

6. Composition pour utilisation selon la revendication 1 ou 2 dans laquelle ledit composé anticancéreux neurotoxique est choisi parmi le bortezomib, la thalidomide ou leur mélange.

7. Composition pour utilisation selon la revendication 2 comprenant entre 1 et 25 mg, de préférence entre 6 et 12 mg, de manière davantage préférée entre 5 à 10 mg de chlorhydrate de donépézil.

8. Composition pour utilisation selon l'une des revendications précédentes **caractérisée en ce que** la composition est administrée par voie orale.

9. Trousse comprenant un composé anticancéreux neurotoxique choisi parmi un sel de platine, le bortézomib, la thalidomide, un poison du fuseau mitotique ou leur mélange ; et un inhibiteur réversible de l'acétylcholinestérase d'action centrale choisi parmi le groupe comprenant le donépézil, un de ses sels pharmaceutiquement acceptable, un de ses énantiomères pharmaceutiquement acceptable.

## Patentansprüche

1. Zusammensetzung zur Verwendung in der Behandlung und/oder Vorbeugung von chemisch induzierten Neuropathien und ihren Symptomen, wobei die Zusammensetzung mindestens einen reversible zentral wirkenden Acetylcholinesterase-Hemmer ausgewählt aus der Gruppe umfassend Donepezil, eines seiner pharmazeutisch unbedenklichen Salze und eines seiner pharmazeutisch unbedenklichen Enantiomere umfasst und die chemisch induzierte Neuropathie eine Neuropathie ist, die von einer Antikrebsverbindung, ausgewählt aus einem Salz von Platin, Bortezomib, Thalidomid, einem Mitosespindelgift oder ihrer Mischung, induziert wird.

2. Zusammensetzung zur Verwendung nach Anspruch 1, wobei es sich bei dem Hemmer um Donepezilhydrochlorid handelt.

3. Zusammensetzung zur Verwendung nach Anspruch 1 oder 2, wobei die neurotoxische Antikrebsverbindung aus den Platinsalzen, vorzugsweise aus der Gruppe umfassend Cisplatin, Carboplatin, Oxaliplatin und ihre Mischung, ausgewählt ist.

4. Zusammensetzung zur Verwendung nach Anspruch 1 oder 2, wobei es sich bei der neurotoxischen Antikrebsverbindung um ein Mitosespindelgift, ausgewählt aus den Alkaloiden des Madagaskar-Immergrüns, den Verbindungen der Epothilonfamilie, den Verbindungen der Taxanfamilie oder ihrer Mischung, handelt.

5. Zusammensetzung zur Verwendung nach Anspruch 4, wobei die neurotoxische Antikrebsverbindung aus den Alkaloiden des Madagaskar-Immergrüns, vorzugsweise aus Vincristin oder ihrer Mischung, ausgewählt ist.

6. Zusammensetzung zur Verwendung nach Anspruch 1 oder 2, wobei die neurotoxische Antikrebsverbindung aus Bortezomib, Thalidomid oder ihrer Mischung ausgewählt ist.

7. Zusammensetzung zur Verwendung nach Anspruch 2, umfassend zwischen 1 und 25 mg, vorzugsweise zwischen 6 und 12 mg, noch stärker bevorzugt zwischen 5 und 10 mg Donepezilhydrochlorid.

8. Zusammensetzung zur Verwendung nach einem der vorhergehenden Ansprüche, **dadurch gekennzeichnet, dass** die Zusammensetzung oral verabreicht wird.

9. Kit, umfassend eine neurotoxische Antikrebsverbindung, ausgewählt aus einem Salz von Platin, Bortezomib, Thalidomid, einem Mitosespindelgift oder ihrer Mischung; und einem reversiblen zentral wirkenden Acetylcholinesterase-Hemmer, ausgewählt aus der Gruppe umfassend Donepezil, eines seiner pharmazeutisch unbedenklichen Salze und eines seiner pharmazeutisch unbedenklichen Enantiomere.

## Claims

1. A composition for use in the treatment and/or prevention of chemically induced neuropathies and their symptoms, said composition comprising at least one reversible centrally acting acetylcholinesterase inhibitor chosen from the group comprising donepezil, a pharmaceutically acceptable salt thereof, a pharmaceutically acceptable enantiomer thereof, and said chemically induced neuropathy being a neuropathy induced by an antineoplastic compound chosen from among a platinum salt, bortezomib, thalidomide, a mitotic spindle poison or a mixture thereof.

2. The composition for use according to claim 1, wherein said inhibitor is donepezil hydrochloride.

3. The composition for use according to claim 1 or 2, wherein said antineoplastic compound is chosen from among the platinum salts; preferably from among the group comprising carboplatin, cisplatin, oxaliplatin and mixtures thereof.

4. The composition for use according to claim 1 or 2, wherein said neurotoxic antineoplastic compound is a mitotic spindle poison chosen from among Madagascar periwinkle alkaloids, compounds in the epothilone class, compounds in the taxane class, or mixtures thereof.

5. The composition for use according to claim 4, wherein said neurotoxic antineoplastic compound is chosen from among Madagascar periwinkle alkaloids; preferably from among vincristine, or mixtures thereof.

6. The composition for use according to claim 1 or 2, wherein said neurotoxic antineoplastic compound is chosen from among bortezomib, thalidomide and mixtures thereof.

7. The composition for use according to claim 2, comprising between 1 and 25 mg, preferably between 6 mg and 12 mg, still more preferably between 5 to 10 mg, of donepezil hydrochloride.

8. The composition for use according to one of the preceding claims, **characterized in that** the composition is administered orally.

9. A kit comprising at least one neurotoxic antineoplastic compound chosen from among a platinum salt, bortezomib, thalidomide, a mitotic spindle poison or a mixture thereof; and a reversible centrally acting acetylcholinesterase inhibitor chosen from among the group comprising donepezil, a pharmaceutically acceptable salt thereof, or a pharmaceutically acceptable enantiomer thereof.
